Europäisches Patentamt
European Patent Office
Office européen des brevets

(19)

(11) Veröffentlichungsnummer: **0 342 182**
**A1**

(12)

# EUROPÄISCHE PATENTANMELDUNG

(21) Anmeldenummer: **89890134.3**

(22) Anmeldetag: **05.05.89**

(51) Int. Cl.4: **C 07 D 405/12**
A 61 K 31/455

(30) Priorität: **13.05.88 AT 1257/88**

(43) Veröffentlichungstag der Anmeldung:
**15.11.89 Patentblatt 89/46**

(84) Benannte Vertragsstaaten:
**BE CH DE ES FR GB GR IT LI LU NL SE**

(71) Anmelder: **Gerot-Pharmazeutika Gesellschaft m.b.H.**
**Arnethgasse 3**
**A-1171 Wien (AT)**

(72) Erfinder: **Schlager, Ludwig H., Dr.**
**Nottebohmstrasse 12**
**A-1190 Wien (AT)**

(74) Vertreter: **Pawloy, Heinrich, Dr. et al**
**Riemergasse 14**
**A-1010 Wien (AT)**

(54) **Neue Derivate von 5-Aminomethyl-2-furanmethanol, ihre Herstellung und Verwendung.**

(57) Es werden neue Derivate von 5-Dialkylaminomethyl-2-furanmethanol der allgemeinen Formel (I)

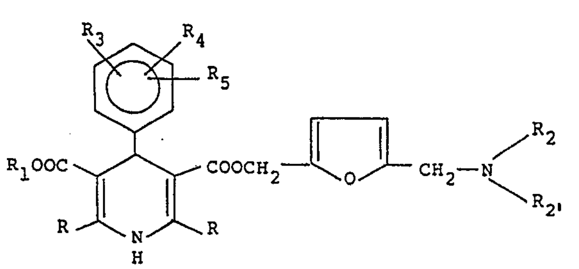

(I) ,

worin R, $R_1$, $R_2$ und $R_2'$ niedere Alkylreste mit 1 bis 4 C-Atomen bedeuten, wobei die Reste $R_2$ und $R_2'$ zusammen mit N auch einen 5- oder 6-gliedrigen gesättigten Heteroring bilden können, der noch ein weiteres Heteroatom wie O, N oder S enthalten kann, und $R_2'$ außer der angegebenen Bedeutung auch Aralkyl darstellen kann, $R_3$, $R_4$ und $R_5$ die in der Beschreibung angegebenen Bedeutungen haben, sowie entsprechende Salze mit physiologisch verträglichen Säuren und ein Verfahren zur Herstellung derselben geoffenbart.

Die neuen Verbindungen besitzen Ca-antagonistische und blutdrucksenkende Wirksamkeit.

EP 0 342 182 A1

**Beschreibung**

**Neue Derivate von 5-Aminomethyl-2-furanmethanol, ihre Herstellung und Verwendung**

Die Erfindung betrifft neue Derivate von 5-Dialkylaminomethyl-2-furanmethanol der allgemeinen Formel (I)

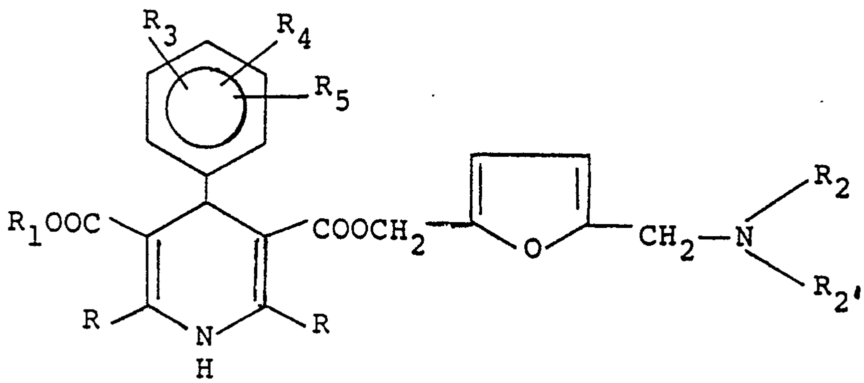

(I) ,

worin R, $R_1$, $R_2$ und $R_2'$ niedere Alkylreste mit 1 bis 4 C-Atomen bedeuten, wobei die Reste $R_2$ und $R_2'$ zusammen mit N auch einen 5- oder 6-gliedrigen gesättigten Heteroring bilden können, der noch ein weiteres Heteroatom wie O, N oder S enthalten kann, und $R_2'$ außer der angegebenen Bedeutung auch Aralkyl darstellen kann, $R_3$ und $R_4$ jeweils Wasserstoff, Halogen, Nitro oder Methoxy bedeuten und $R_5$ Halogen, Difluormethoxy, Trifluormethyl, Nitro, Carbamylmethoxy, Cyanmethoxy oder Mesyloxy ist, sowie deren Salze mit physiologisch verträglichen Säuren.

Das einfachste 5-Dialkylaminomethyl-2-furanmethanol der Formel

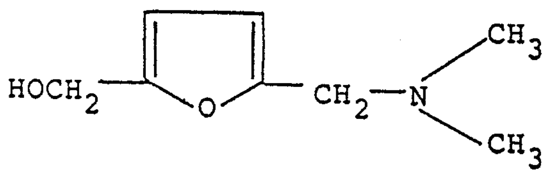

(II)

ist zwar schon seit 1947 bekannt, wurde aber erst 1976 durch die Entdeckung des Antiulcus-Wirkstoffes Ranitidin zu Arzneimittel-Synthesen herangezogen, die sich seither sowohl auf das vorgegebene Indikationsgebiet als auch zumeist auf die Verwendung von (II) zur Herstellung der entsprechenden Furanmethylthioäther beschränkten.

Von Estern des Alkohols (II) sind bisher nur solche mit Schwefelsäure bekannt, die aber ebenfalls als Antiulcusmittel beschrieben sind.

Bei dieser Fixierung der (II)-Struktur auf die Antiulcus-Indikation war nicht vorherzusehen, daß neue Ester von (II) kreislaufwirksame - insbesondere calciumantagonistische und antihypertensive - Eigenschaften aufweisen würden.

Es gibt zwar bereits 1,4-Dihydropyridin-3,5-dicarbonsäureester, die im Esterrest Furanringe enthalten (Chem.Abstr. 75, 151687 d; 100, 85591 z), aber nicht mit solchen Substituenten, wie sie in den Formeln (I) und (II) dargestellt sind.

Um die Chancen zur pharmakologischen Verwendbarkeit von Verbindungen der allgemeinen Formel (I) zu ermitteln, wurde zunächst die akute Toxizität des Hydrochlorids von (II) untersucht, da dieses beim Metabolismus von (I) gebildet werden kann. Dieses (II)-Hydrochlorid zeigt mit einer $LD_{50}$ von 289 mg/kg (Maus, i.v.) eine sehr geringe Toxizität.

Das aus (II) gemäß Beispiel 1 erhältliche Endprodukt wirkt aber sowohl im pharmakologischen Test auf Ca-Antagonismus (Tonus des Rattenuterus im Organbad) als auch bezüglich der Blutdrucksenkung der Ratte stärker als die bekannten Vergleichspräparate Nifedipin und Verapamil.

Zur Herstellung der erfindungsgemäßen Verbindungen setzt man Derivate von 5-Aminomethyl-2-furanmethanol der allgemeinen Formel (III)

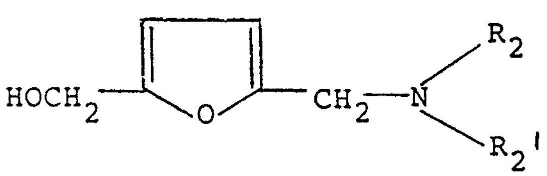

(III) ,

worin $R_2$ und $R_2'$ die oben genannten Bedeutungen haben, mit Diketen oder dessen Acetonaddukt um, kondensiert die erhaltene neue 2-Acetoacetoxymethylverbindung der allgemeinen Formel (IV)

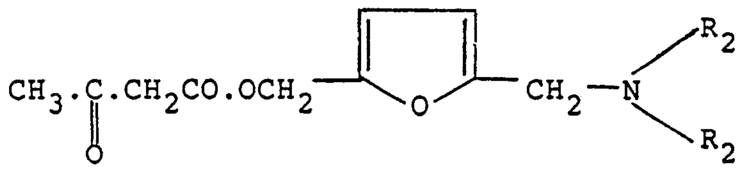

(IV)

mit einem substituierten Benzaldehyd und cyclisiert das Reaktionsprodukt mit einemn 3-Aminocrotonsäureester zum Endprodukt der allgemeinen Formel (I).

Die neuen Wirkstoffe der allgemeinen Formel (I) können entweder allein oder zusammen mit pharmazeutisch verwendbarem Trägermaterial gegen Bluthochdruck gegeben werden. Die galenische Verarbeitung zu Tabletten, Kapseln, Lösungen, Suspensionen oder Suppositorien erfolgt in der dazu jeweils geeigneten Weise unter Verwendung der dem Fachmann an sich bekannten Hilfsstoffe.

Zur Herabsetzung des Blutdrucks von Hypertonikern wird die Dosierung dem vorgegebenen Überdruck angepaßt und mit relativ geringen Dosen begonnen. Im allgemeinen wird man pro Applikation mit 1 bis 100 mg Wirkstoff auskommen.

Durch die folgenden Beispiele soll die Erfindung näher erläutert, aber nicht auf diese beschränkt werden.

**Beispiel 1:**

Eine Mischung von 54,7 g 5-Dimethylaminomethyl-2-acetoacetoxymethylfuran und 40 g 2,3-Dichlorbenzaldehyd wird nach Zusatz von je 8 Tropfen Eisessig und Piperidin in 200 ml trockenem Methylenchlorid unter einem Wasserabscheider bis zur Beendigung der Wasserbildung erhitzt. Man filtriert das Reaktionsgemisch unter Verwendung von etwas Aktivkohle und Celite und dampft das Filtrat ein.

Der Rückstand wird mit 26,3 g 3-Aminocrotonsäuremethylester in 150 ml absol. Äthanol 6 h unter Rückfluß erhitzt. Der Eindampfrückstand dieser Reaktion wird in 50 ml Äthylacetat aufgenommen und durch Säulenchromatographie an 300 g Kieselgel 60 gereinigt, wobei als Eluiermittel Äthylacetat verwendet wird. Die Reinfraktion wird eingedampft, das erhaltene Öl in Aceton gelöst und mit alkoholischer HCl angesäuert. Das ausgefallene Rohprodukt wird durch Auskochen mit Isopropyläther und durch Umkristallisieren aus Aceton weiter gereinigt. Das so erhaltene Hydrochlorid von 2,6-Dimethyl-3-(5-dimethylaminomethylfuran-2-methoxycarbonyl)-4-(2,3-dichlorphenyl)-5-methoxycarbonyl-1,4-dihydropyridin schmilzt bei 189 - 191°C.

Die als neues Ausgangsprodukt eingesetzte Furankomponente wird folgendermaßen hergestellt: 89 g frisch destilliertes 5-Dimethylaminomethyl-2-furanmethanol und 84,6 g Diketenacetonaddukt erhitzt man in 80 ml Toluol 1 h unter Rückfluß und läßt das allmählich frei werdende Aceton abdestillieren. Den Eindampfrückstand nimmt man in Äthylacetat auf und reinigt die Lösung über eine kurze Säule mit Kieselgel 60. Das nach dem Eindampfen des Eluates zurückbleibende Öl besteht aus 5-Dimethylaminomethyl-2-acetoacetoxymethylfuran und kann zur Kondensation mit substituierten Benzaldehyden eingesetzt werden.

**Beispiel 2:**

2,7 g des gemäß Beispiel 1 erhältlichen Ausgangsprodukts (Formel IV), $R_2$ = Methyl) werden in 80 ml absol. Benzol in Anwesenheit von 0,3 g Piperidinacetat mit 2,0 g 2-Carbamylmethoxybenzaldehyd unter Rückfluß und Wasserabscheidung erhitzt. Nach 2 h dampft man das Reaktionsgemisch ein, nimmt den Rückstand in 20 ml absol. Methanol auf, versetzt mit 1,3 g 3-Aminocrotonsäuremethylester und kocht 1 1/2 h unter Rückfluß weiter. Der Eindampfrückstand wird nun in Äthylacetat mit Wasser gewaschen, die organische Lösung mit 2 n Essigsäure extrahiert, die wässerige Phase mit verdünnter NaOH neutralisiert und mit Äthylacetat ausgeschüttelt. Nach dem Eindampfen der erhaltenen Lösung bleibt das Rohprodukt als orangefarbiges Öl zurück, das durch Säulenchromatographie an Kieselgel 60 unter Verwendung von Aceton gereinigt wird. Das Endprodukt 2,6-Dimethyl-3-(5-dimethylamino methylfuran-2-methoxycarbonyl)-4-(2-carbamylmethoxyphenyl)-5methoxycarbonyl-1,4-dihydropyridin kristallisiert als Base aus Äthylacetat beim Stehen in der Kälte und schmilzt bei 174 -175°C.

**Beispiel 3:**

Wird das gemäß Beispiel 1 erhältliche Ausgangsprodukt (Formel (IV), $R_2$ = Methyl) mit 2-Chlorbenzaldehyd umgesetzt und im übrigen nach den Beispielen 1 oder 2 verfahren, dann erhält man das 2,6-Dimethyl-3-(5-dimethylaminomethylfuran-2-methoxycarbonyl)-4-(2-chlorphenyl)-5-methoxycarbonyl-1,4-dihydropyridin als eine bei 154 - 156°C schmelzende Base.

**Beispiel 4:**

Eines der Ausgangsprodukte wird folgendermaßen hergestellt: Zu einer Lösung von 10 g 3,5-Dibromsalicylaldehyd in 100 ml Methanol tropft man unter Rühren 6,43 g einer 30 %igen methanolischen Lösung von Natriummethylat. Der Eindampfrückstand dieser Phenolatlösung wird in 50 ml Dimethylformamid aufgenommen und nach Zusatz von 2,68 g Chloracetonitril, 1 g Triäthylbenzylammoniumchlorid und 1 g Kaliumjodid 5 h auf 40°C erwämt. Danach dampft man die Mischung im Vakuum ein, nimmt den Rückstand in Äthylacetat auf, schüttelt die Lösung nacheinander mit Wasser, 1n NaOH, 1n HCl und Wasser aus, filtriert nach Zusatz von Aktivkohle und dampft das Filtrat ein. Durch Aufkochen des Rohproduktes in Isopropyläther erhält man 2-Cyanmethoxy-3,5-dibrombenzaldehyd als bräunliches Pulver, das bei 125 - 130°C schmilzt.

Setzt man dieses neue Salicylaldehydderivat an Stelle der in den Beispielen 1 und 2 verwendeten

Benzaldehydverbindungen ein und verfährt ansonsten wie in den obigen Beispielen angegeben, dann erhält man das 2,6-Dimethyl-3-(5 dimethylaminomethylfuran-2-methoxycarbonyl)-4-(2-cyanmethoxy 3,5-dibromphenyl)-5-methoxycarbonyl-1,4-dihydropyridin, das bei 145 - 150°C schmilzt.

**Beispiel 5:**

Als Ausgangsprodukt wird hier 2-(N-Diphenylmethylcarbamylmethoxy)-benzaldehyd verwendet, der folgendermaßen hergestellt wrid:

Eine Lösung von 1,41 g Salicylaldehyd in 10 ml Methanol wird mit 2,08 g einer 30 %igen methanolischen Lösung von Natriummethylat versetzt. Der Eindampfrückstand wird in 25 ml absol. n-Propanol aufgenommen und mit 3 g N-(Diphenylmethyl)-chloracetamid (Fp: 131 - 134°C, aus α-Aminodiphenylmethan und Chloracetylchlorid in Äthylacetat/$NaHCO_3$) 12 h unter Rückfluß gerührt. Nach Abdampfen des Lösungsmittels löst man den Rückstand in heißem Äthylacetat, filtriert und verdünnt das kalte Filtrat mit Isopropyläther. Beim Stehen in der Kälte kristallisiert 2-(N-Diphenylmethylcarbamylmethoxy)-benzaldehyd, der bei 110 - 113°C schmilzt.

Wird dieser Aldehyd in der in den Beispielen 1 oder 2 beschriebenen Weise umgesetzt, dann wird 2,6-Dimethyl-3-(5-dimethylaminomethylfuran-2-methoxycarbonyl)-4-[2-(N-diphenylmethylcarbamylmethoxy)-phenyl]-5-methoxycarbonyl-1,4-dihydropyridin mit Fp. 163 - 164°C erhalten.

**Beispiel 6 :**

5 g 5-(Piperidinomethyl)-furfurylalkohol (Chem.Abstr. 69, 59003f (1968)) werden mit 3,8 g Diketenacetonaddukt in 20 ml absol. Toluol unter Rückfluß erhitzt, bis kein Aceton mehr abdampft. Durch Eindampfen der Reaktionsmischung im Vakuum verbleibt ein orangefarbiges Öl, das aus 2-Acetoacetoxymethyl-5-piperidinomethylfuran besteht.

3,1 g dieses Öls erhitzt man mit 2 g 2-Carbamylmethoxybenzaldehyd in 80 ml absol. Benzol unter Zusatz von je 2 Tropfen Eisessig und Piperidin etwa 2 h unter Rückfluß und Wasserabscheidung. Der Eindampfrückstand wird dann in 20 ml absol. Methanol gelöst und nach Zusatz von 1,3 g 3-Aminocrotonsäuremethylester etwa 2 h unter Rückfluß gerührt.

Der Verlauf der Kondensations- und Cyclisierungsreaktion ist am Dünnschichtchromatogramm (Kieselgel 60F 254, Laufmittel: Chloroform/Methanol = 9 : 1) zu verfolgen.

Nach Beendigung der Reaktion wird das Lösungsmittel abdestilliert, der Rückstand in Äthylacetat aufgenommen und die Lösung nacheinander mit Wasser und 5n HCl ausgeschüttelt. Der saure Extrakt wird mit 5n NaOH neutralisiert und die Mischung wieder mit Äthylacetat extrahiert. Nach dem Trocknen (mit $Na_2SO_4$) und Eindampfen der organischen Lösung verbleibt ein gelber Rückstand, der aus Aceton umkristallisiert bei 180 - 182°C schmilzt und aus 2,6-Dimethyl-3-(5-piperidinomethylfuran-2-methoxycarbonyl)-4-(2-carbamylmethoxyphenyl)-5-methoxycarbonyl-1,4-dihydropyridin besteht.

Entsprechend den Beispielen 1 bis 6 sind weitere neue Derivate der allgemeinen Formel (I) erhältlich, wie aus nachstehender Tabelle hervorgeht:

| R | $R_1$ | 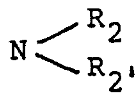 | $R_3$ | $R_4$ | $R_5$ | Fp°C (Base/Salz) |
|---|---|---|---|---|---|---|
| $CH_3$ | $CH_3$ | $N(CH_3)_2$ | H | H | 2-O.$SO_2CH_3$ | 100-105 (.HCl) |
| $CH_3$ | $CH_3$ | 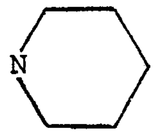 | H | H | 2-F | 150-153 (Base) |
| $CH_3$ | $CH_3$ | $N(CH_3)_2$ | H | H | 2-$CF_3$ | 116-118 (Base) |
| $CH_3$ | $CH_3$ | $N(CH_3)_2$ | H | H | 2-O.$CH_2CON(CH_3)_2$ | 109-113 (.HCl) |
| $CH_3$ | $CH_3$ | $N(CH_3)_2$ | H | H | 2-$NO_2$ | 106-108 (Base) |
| $CH_3$ | $CH_3$ | 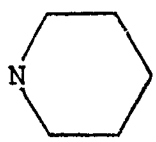 | H | 3-Cl | 2-Cl | 84-85 (Base) |
| $CH_3$ | $CH_3$ | $N(CH_3)_2$ | H | H | 2-O.$CH_2$CO.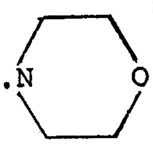 | 147-155 (Base) |
| $CH_3$ | $CH_3$ | 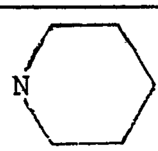 | H | H | 2-Cl | 137 (Base) |

| R | $R_1$ | 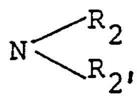 | $R_3$ | $R_4$ | $R_5$ | Fp°C (Base/Salz) |
|---|---|---|---|---|---|---|
| $CH_3$ | $CH_3$ | 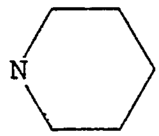 | H | H | 2-$CF_3$ | 152-155 (Base) |
| $CH_3$ | $CH_3$ | 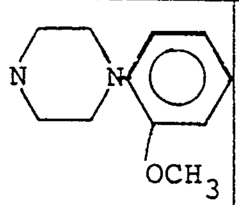 | H | H | 2-O.$CH_2CONH_2$ | 170-175 (Base) |
| $CH_3$ | $CH_3$ | 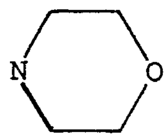 | H | H | 2-O.$CH_2CONH_2$ | 179-180 (Base) |
| $CH_3$ | $CH_3$ | $N(C_2H_5)_2$ | H | H | 2-O.$CH_2CONH_2$ | 152-153 (Base) |
| $CH_3$ | $CH_3$ | $N(CH_3)_2$ | 4-F | H | 2-F | 111-113 (Base) |
| $CH_3$ | $CH_3$ | 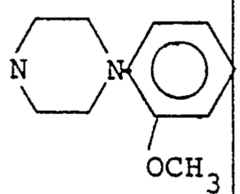 | H | H | 3-$NO_2$ | 141-143 (Base) |
| $CH_3$ | $CH_3$ | $N(CH_3)_2$ | H | H | 3-$NO_2$ | 136-138 (Base) |
| $CH_3$ | $CH_3$ | 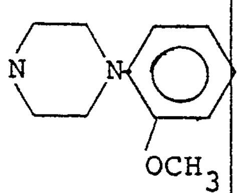 | H | 3-Cl | 2-Cl | 144-146 (Base) |

| R | $R_1$ | 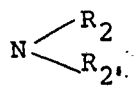 | $R_3$ | $R_4$ | $R_5$ | Fp°C (Base/Salz) |
|---|---|---|---|---|---|---|
| $CH_3$ | $CH_3$ | 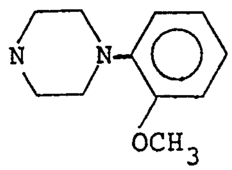 | H | H | 2-F | 117-120 (Base) |
| $CH_3$ | $CH_3$ | 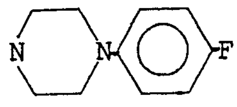 | H | H | $2-OCH_2CONH_2$ | > 200 Zers. (Base) |
| $CH_3$ | $CH_3$ | 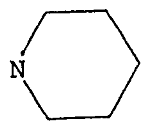 | H | H | $2-O.SO_2CH_3$ | 95-101 (.HCl) |
| $CH_3$ | $CH_3$ | 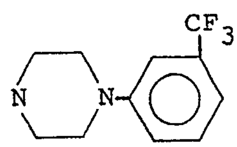 | H | H | $2-O.CH_2CONH_2$ | 185-190 (Base) |
| $CH_3$ | $CH_3$ | 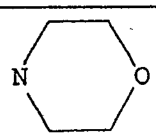 | H | H | 2-F | 169-171 (Base) |
| $CH_3$ | $CH_3$ | $N(CH_3)_2$ | H | H | 2-F | 103-105 (Base) |
| $CH_3$ | $CH_3$ | 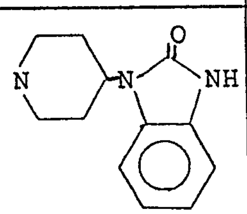 | H | H | 2-Cl | 95-118 (Base) |

**Beispiel 7:**

Der als neues Zwischenprodukt verwendete 5-[4-(2-Methoxyphenyl)-piperazinomethyl]-furfurylalkohol wurde durch Mannich-Kondensation hergestellt:

Eine Mischung von 30 g Furfurylalkohol, 70 g N-(2-Methoxyphenyl)-piperazinhydrochlorid und 23 g p-Formaldehyd wurde in 200 ml Äthanol 12 h unter Rückfluß gerührt, danach im Vakuum eingedampft und der Rückstand mit wenig Wasser verdünnt. Das durch Zusatz von NaOH alkalisch gemachte Konzentrat wurde

mehrmals mit Diäthyläther extrahiert, die Ätherlösung mit Aktivkohle und $Na_2SO_4$ sicc. behandelt, filtriert und eingedampft. Eine Lösung des Rückstandes in Äthylacetat wurde durch Säulenchromatrographie mit Kieselgel 60 gereinigt. Die Reinfraktion ergab nach dem Eindampfen ein Öl, das beim Stehen kristallisierte. Nach dem Waschen mit Diisopropyläther wurde ein bei 97 - 99°C schmelzendes Pulver erhalten.

**Beispiel 8:**

Das als neues Zwischenprodukt verwendete 5-[4-(2-Oxo-1-benzimidazolinyl)-piperidinomethyl]-2-furanmethanol wurde entsprechend der Verfahrensweise von Beispiel 7 durch Mannich-Reaktion von 4-(2-Oxo-1-benzimidazolinyl)-piperidinhydrochlorid, Furfurylalkohol und p-Formaldehyd in siedendem Äthanol erhalten. Das Zwischenprodukt zerfließt nach Reinigung über eine Kieselgel 60-Säule ab 73°C.

**Patentansprüche**

1. Neue Derivate von 5-Dialkylaminomethyl-2-furanmethanol der allgemeinen Formel (I)

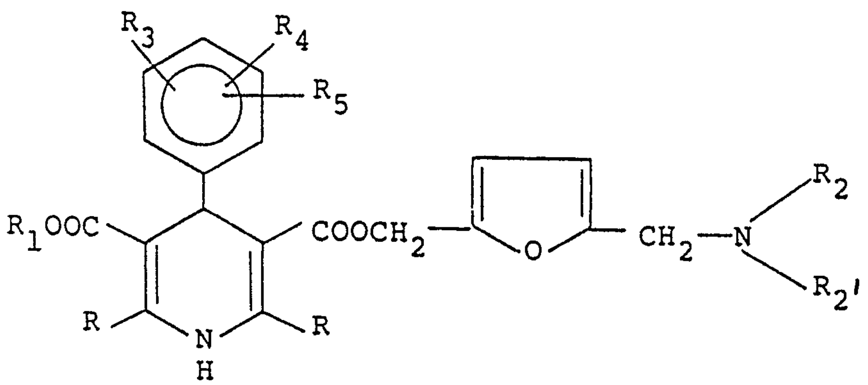

(I) ,

worin R, $R_1$, $R_2$ und $R_2'$ niedere Alkylreste mit 1 bis 4 C-Atomen bedeuten, wobei die Reste $R_2$ und $R_2'$ zusammen mit N auch einen 5- oder 6-gliedrigen gesättigten Heteroring bilden können, der noch ein weiteres Heteroatom wie O, N oder S enthalten kann, und $R_2'$ außer der angegebenen Bedeutung auch Aralkyl darstellen kann, $R_3$ und $R_4$ jeweils Wasserstoff, Halogen, Nitro oder Methoxy bedeuten und $R_5$ Halogen, Difluormethoxy, Trifluormethyl, Nitro, Carbamylmethoxy, Cyanmethoxy oder Mesyloxy ist, sowie deren Salze mit physiologisch verträglichen Säuren.

2. Verfahren zur Herstellung von Verbindungen der allgemeinen Formel (I), wie in Anspruch 1 definiert, und deren Salzen mit physiologisch verträglichen Säuren, dadurch gekennzeichnet, daß man ein 5-Aminomethyl-2-furanmethanol der allgemeinen Formel (III)

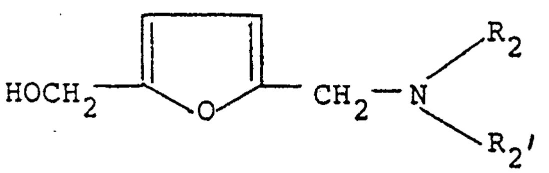

(III) ,

worin $R_2$ und $R_2'$ die in Anspruch 1 genannten Bedeutungen haben, mit Diketen oder dessen Acetonaddukt umsetzt, die erhaltene neue 2-Acetoacetoxymethylverbindung der allgemeinen Formel (IV)

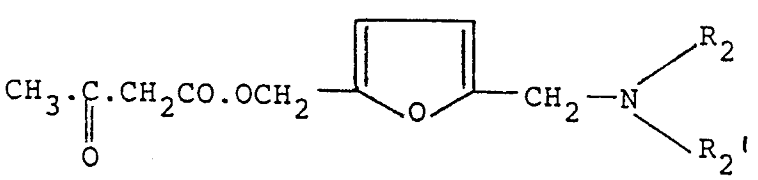

(IV)

worin $R_2$ und $R_2'$ die obigen Bedeutungen haben, vorzugsweise in Anwesenheit eines Katalysators, wie z.B. Piperidinacetat, mit einem substituierten Benzaldehyd unter Wasserabscheidung kondensiert, das Reaktionsprodukt mit einem 3-Aminocrotonsäureester zum Endprodukt der allgemeinen Formel (I) cyclisiert und gegebenenfalls dieses mit einer physiologisch verträglichen Säure in ein Salz überführt.

3. Verwendung von Verbindungen der allgemeinen Formel (I), wie in Anspruch 1 definiert, zur Herstellung von Medikamenten zur Behandlung von Bluthochdruck.

Europäisches Patentamt

# EUROPÄISCHER RECHERCHENBERICHT

Nummer der Anmeldung

EP 89890134.3

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A2 - 0 088 903 (YOSHITOMI)<br>* Zusammenfassung *<br>-- | 1,3 | C 07 D 405/12<br>A 61 K 31/455 |
| A | DE - A1 - 2 003 148 (BAYER)<br>* Ansprüche 1,2 *<br>-- | 1,3 | |
| A | CHEMICAL ABSTRACTS, Band 94, Nr. 19, 11. Mai 1981, Columbus, Ohio, USA<br>GONCHAROVA R.I. et al. "Comparative study of the antimutagenic action of dihydropyridine series compounds in connection with their antioxidant activity."<br>Seite 142, Spalte 2, Zusammenfassung Nr. 151 029h<br>& Dokl.Akad.Nauk SSSR 1980, 255(6), 1483-6<br>-- | 1 | |
| | | | RECHERCHIERTE SACHGEBIETE (Int. Cl.4) |
| A | CHEMICAL ABSTRACTS, Band 84, Nr. 3, 19. Jänner 1976, Columbus, Ohio, USA<br>ULDRIKJIS; J. et al. "Synthesis and oxidation of 2,6-dimethyl-1,4-dihydropyridine-3,5-carboxylates."<br>Seite 443, Spalte 1, Zusammenfassung Nr. 17 090m<br>& Khim.Geterotsikl.Soedin 1975, (9), 1230-7<br>-- | 1 | C 07 D 405/00 |
| A | CHEMICAL ABSTRACTS, Band 70, Nr. 19, 12. Mai 1969, Columbus, Ohio, USA<br>BOSSERT, FRIEDRICH et al. "1,4-Dihydropyridine-3,5-dicarboxylates." | 1 | |

Der vorliegende Recherchenbericht wurde für alle Patentansprüche erstellt.

| Recherchenort | Abschlußdatum der Recherche | Prüfer |
|---|---|---|
| WIEN | 03-08-1989 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-
EP 89890134.3

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| | Seite 316, Spalte 1, Zusammenfassung Nr. 87 580f<br>& S. African 68 01,484<br>-- | | |
| A | EP - A2 - 0 194 906<br>(SOCIETE DE RECHERCHES)<br>* Zusammenfassung *<br>-- | 1,3 | |
| A | EP - B1 - 0 068 171<br>(YOSHITOMI)<br>* Formel I, Anspruch 10 *<br>-- | 1,3 | |
| A | CHEMICAL ABSTRACTS, Band 101, Nr. 13, 24. September 1984, Columbus, Ohio, USA<br>YOSHITOMI PHARMACEUTICAL INDUSTRIES, LTD. "1,4-Dihydropyridine-3,5-dicarboxylic acid ester derivatives."<br>Seite 640, Spalte 1, Zusammenfassung Nr. 110 746j<br>& Jpn. Kokai Tokky Koho JP 59 51,281 /84 51,281/<br>-- | 1 | RECHERCHIERTE SACHGEBIETE (Int. Cl.4) |
| A | CHEMICAL ABSTRACTS, Band 101, Nr. 17, 22. Oktober 1984, Columbus, Ohio, USA<br>YOSHITOMI PHARMACEUTICAL INDUSTRIES, LTD. "1,4-Dihydropyridine-3,5-dicarboxylic acid ester derivatives."<br>Seite 718, Spalte 1, Zusammenfassung Nr. 151 862n<br>& Jpn. Kokai Tokkyo Koho JP 59 78,186 /84 78,186/<br>-- | | |
| A | CHEMICAL ABSTRACTS, Band 98, Nr. 25, 20. Juni 1983, Columbus, Ohio, USA<br>YOSHITOMI PHARMACEUTICAL | | |

Der vorliegende Recherchenbericht wurde für alle Patentansprüche erstellt.

| Recherchenort | Abschlußdatum der Recherche | Prüfer |
|---|---|---|
| WIEN | 03-08-1989 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | INDUSTRIES, LTD. "1,4-Dihydropyridine-3,5-dicarboxylic acid esters." Seite 550, Spalte 1, Zusammenfassung Nr. 215 490u & Jpn. Kokai Tokkyo Koho JP 57,200 386 /82,200,386/ ---- | | |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| WIEN | 03-08-1989 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument